# EUROPÄISCHE PATENTSCHRIFT

(11) **EP 1 811 978 B1**
(45) Veröffentlichungstag und Bekanntmachung des Hinweises auf die Patenterteilung: **23.03.2011**
(21) Anmeldenummer: 05786032.2
(22) Anmeldetag: 22.09.2005
(51) Int. Cl.: A61K 9/50

(54) **HERSTELLUNG VON ZWEI- ODER MEHRSCHICHTIG AUFGEBAUTEN MIKROKAPSELN**
PRODUCTION OF DOUBLE- OR MULTI-LAYERED MICROCAPSULES
PRODUCTION DE MICROCAPSULES A DEUX OU PLUSIEURS COUCHES

(30) Priorität: 18.11.2004 DE 102004055729
(43) Veröffentlichungstag der Anmeldung: 01.08.2007
(73) Patentinhaber: CellMed AG, 63755 Alzenau (DE)
(72) Erfinder: THOENES, Eric, 63654 Büdingen (DE); GEIGLE, Peter, 63755 Alzenau (DE)
(74) Vertreter: Graf von Stosch, Andreas
(86) Internationale Anmeldenummer: PCT/EP2005/010277
(87) Internationale Veröffentlichungsnummer: WO 2006/053604

(56) Entgegenhaltungen:
- WO-A-98/23226
- US-A1- 2002 022 016
- JORK A ET AL: "Biocompatible alginate from freshly collected Laminaria pallida for implantation" APPLIED MICROBIOLOGY AND BIOTECHNOLOGY, Bd. 53, Nr. 2, Februar 2000 (2000-02), Seiten 224-229, XP002368029 ISSN: 0175-7598 in der Anmeldung erwähnt
- CLAYTON H A: "THE EFFECT OF CAPSULE COMPOSITION ON THE BIOCOMPATIBILITY OF ALGINATE-POLY-L-LYSINE CAPSULES" JOURNAL OF MICROENCAPSULATION, TAYLOR AND FRANCIS, BASINGSTOKE, GB, Bd. 8, Nr. 2, 1. April 1991 (1991-04-01), Seiten 221-233, XP000216619 ISSN: 0265-2048 in der Anmeldung erwähnt

## Beschreibung

### Gebiet der Erfindung

Die Erfindung bezieht sich auf ein Verfahren zur Herstellung zwei- oder mehrschichtig aufgebauter sphärischer Mikrokapseln wie in Anspruch 1, 2 und 7 definiert.

US 2002/022016 offenbart ein Verfahren zur Verkapselung von biologischem Material, wobei in einem ersten Schritt aus einer Suspension von Alginat und biologischem Material Tropfen geformt werden. Diesen Tropfen werden mit einer zweiten Schicht aus einer Alginat und Suspension umhüllt.

WO 98/23226 offenbart ein Verfahren zur Herstellung von mehrschichtig aufgebauten Mikrokapseln, wobei ein Kern, der biologisches Gewebe enthält, mit einer ersten Schicht aus Alginat umgeben wird, worauf sich eine Schicht aus z. B. Poly-L-Lysin und abschließend eine weitere Schicht aus Alginat anschließt.

Jork A. et. al. beschreiben in Applied Microbiology and Biotechnoloy, Band 53, Nr. 2 (2002), Seite 224-229 die Verkapselung von Knorpelzellen in einem zweilagigen Alginatkügelchen Die Kügelchen bestehen aus einem Kern mit Knorpelzellen und vernetztem Alginat, der von einer Schicht aus vernetztem Alginat umhüllt ist.

Clayton beschreibt in Journal of Microencapsulation, Band 8, Nr. 2 (1991), Seite 221-233, die Biokompatibilität von aus Alginat bestehenden Mikrokapseln. Die Mikrokapseln enthalten entweder keine weitere Schicht oder eine zusätzliche Schicht aus N-Alginat E-Alginat.

### Hintergrund der Erfindung

In der Medizin ist bei allogenen oder xenogenen Transplantationen bekannt, dass Immunreaktionen des Wirtsorganismus durch eine Mikroverkapselung des Transplantates (biologische Zellen oder biologisches Gewebe) in Polymeren zu dessen Immunisolation zu begegnen ist. (Lim F and Sun AM in "Science", Bd. 210, 1980, S. 908-910; Stevenson WTK and Sefton MV. 1992, in "Fundamentals of Animal Cell Encapsulation and Immobilization", MFA Goosen, ed., CRC Press, Boca Raton, FI.).

Bei diesen Verfahren werden körperfremde biologische Zellen (ggf. gentechnisch modifiziert) oder körperfremde biologische Gewebe in eine Matrix vorzugsweise aus Polymeren durch verschiedenste Vertropfungsverfahren verkapselt (immobilisiert) und in den Patienten transplantiert. Diese Matrix muss einerseits durchlässig für die Versorgung der Zellen mit Sauerstoff und Nährstoffen sein, andererseits die Diffusion des therapeutischen Proteins aus der Kapsel heraus in den Patienten ermöglichen. Hingegen dürfen Komponenten des körpereigenen Immunsystems nicht durch die Matrix gelangen. Zur Anwendung als Matrixmaterial kommen hierbei synthetische, semi-synthetische und natürliche, wasserlösliche Biopolymere (z.B. Alginate, Hyaluronsäure, Cellulosesulphate etc.). Aufgrund seiner Biokompatibilität und seiner Vernetzungseigenschaften eignen sich vorzugsweise Alginate für diese Anwendung. Chemisch gesehen sind Alginate anionische Polysaccharide aus homopolymeren Gruppen von β-D-Mannuronsäure und α-L-Guluronsäure, getrennt durch heteropolymere Regionen beider Säuren. Im Beisein von monovalenten Kationen, wie zum Beispiel Natrium oder Kalium, sind Alginate wasserlöslich und bilden hochviskose Lösungen. Durch Wechselwirkung der einzelnen Alginatketten mit di-, tri- oder multivalenten Kationen (wie Calcium, Barium oder Polylysin) entsteht ein quervernetztes wasserunlösliches Hydrogel.

Bei der Herstellung der sphärischen Mikrokapseln ist es von entscheidender Bedeutung, dass die eingekapselten Zellen oder Gewebe vollständig in die Polymermatrix eingebettet sind. Sehr nahe am Rand liegende Zellen/Gewebe oder aus der Matrix herausstehende Zellen oder Gewebeteile würden das Prinzip der Immunisolation außer Kraft setzen, da die Immunabwehr diese körperfremden Komponenten erkennen und das Transplantat zerstören würde. Bei einschichtigen Mikrokapseln kann das nur vermieden werden, in dem die Konzentration der Zellen in der zu vertropfenden Suspension sehr niedrig gehalten wird, um die Wahrscheinlichkeit randständiger Zellen zu vermeiden. Um aber einen möglichst hohen Wirkstofflevel zu erzielen und das Transplantatgesamtvolumen möglichst gering zu halten, ist es meist notwendig, sehr hohe Konzentrationen an biologischen Zellen zu verkapseln. Daher ist es vorteilhaft, um die einschichtige Mikrokapsel eine weitere Hüllschicht aus einem reinem Polymer aufzutragen. In der Literatur sind hierfür sogenannte Dreikanaldüsen vorgeschlagen (Jork et al. in "Appl Microbiol Biotechnol" Bd. 53, 2000, S. 224-229, US 09/762,850). Durch den inneren Kanal wird dabei das Poiymer/Zellgemisch gedrückt, durch den zweiten Kanal das reine Polymer ohne biologische Zellen, und durch den äußeren Kanal Druckluft, die das Abreißen der Tropfen bewirkt. Dabei wird in einem Verfahrensschritt eine Polymerkapsel mit einem Kern aus Polymer und biologischen Zellen und einer äußeren Hüllschicht ohne biologische Zellen hergestellt. Die Vernetzung des Polymers im Kern und in der Hüllschicht findet gleichzeitig statt. Der Nachteil dieses Verfahrens ist, dass durch die sofortige Umhüllung der Kern nicht auf seine gewünschte sphärische Form abrunden kann sondern eine eher spindelähnliche Form mit zwei langgezogenen Enden erhält. Je visköser das eingesetzte Polymer ist, desto mehr verstärkt sich dieser Effekt und die Gefahr randständiger oder an der Oberfläche liegender Zellen bleibt durch die Form des langgezogenen Kernes bestehen. Weiterhin nachteilig an diesem Verfahren ist, dass durch die Spindelform des inneren Kerns die Hüllschicht keine einheitliche Dicke aufweist, sondern an den Polen der Spindel dünner (oder gar nicht vorhanden) ist, während sie am Äquator der Kapseln dicker ist. Dadurch ergeben sich unterschiedlich lange Diffusionsstrecken für Moleküle, z.B. für Sauerstoff, Nährstoffe oder Proteine. Dies kann eine unterschiedliche Versorgung oder sogar ein Absterben der enkapsulierten Zellen oder ein zeitlich ungleichmäßiges Ausschleusen des durch die verkapselten Zellen produzierten therapeutisch wirksamen Proteins nach sich ziehen. Aus der ungünstigen geometrischen Form ergibt sich außerdem ein großes Verhältnis von Gesamtvolumen zu Kernvolumen. Dies bedeutet, dass ein großer Teil des zur Verfügung stehenden Gesamtvolumens nicht optimal für die Verkapselung von Zellen genutzt werden kann und daher die Konzentration an biologischen Zellen im Vergleich zum Gesamtvolumen gering ist. Um aber dennoch auf eine ausreichende Anzahl an zu verkapselnden Zellen zu kommen, muss unnötiger Weise das Gesamttransplantatvolumen erhöht werden.

Eine andere aus der Literatur bekannte Möglichkeit die einschichtigen Kapseln mit einer zusätzlichen Hüllschicht zu umgeben ist die Vernetzung mit Polykationen, wie z.B. Poly-L-Lysin. Dabei werden nach dem Stand der Technik einschichtige biologische Zellen enthaltene Mikrokapseln z.B. aus Alginat hergestellt und in einer Poly-L-Lysin enthaltenen Lösung inkubiert. Durch die Bindung von Poly-L-Lysin an das Alginat kommt es zur Ausbildung einer sog. Polyanion-Polykation-Membran. Anschließend werden diese Kapseln wieder in einer Lösung aus anionischem Polymer (z.B. Alginat) getaucht, welches sich wiederum ionisch an die Poly-L-Lysin Schicht bindet. Nachteilig an den Alginat-Poly-L-Lysin-Alginat Kapseln ist jedoch eine verstärkte Immunantwort des Transptantatempfängers aufgrund des Polykations, zudem erwies sich das Poly-L-Lysin als zytotoxisch für die verkapselten Zellen (King et al. in "J Biomed Mater Res.", Bd. 57, 2001, S. 374-383; Strand et al. in "Cell Transplant.", Bd. 10, 2001, S. 263-275; De Vos et al. in "Biomaterials", Bd. 18, 1997, S. 273-278). Zudem ist die Dicke der äußeren Hüllschicht durch dieses Verfahren sehr gering (ionisch vernetzter Monolayer) und nicht variabel einstellbar.

### Zusammenfassung der Erfindung

Die vorliegende Erfindung, beschreibt ein Verfahren zur Herstellung zwei- oder mehrschichtig aufgebauter sphätischer Mikrokapseln wie in Anspruch 1, 2 und 7 definiert.

Durch diese Erfindung wird vorteilsmäßig ein sphärischer Kern aus vernetztern Biopolymer und biologischen Zellen erreicht. Der Kern ist durch eine homogene Verteilung der biologischen Zellen und eine gleichmäßige Dicke der äußeren Hüllschicht gekennzeichnet. Ein weiterer Vorteil der vorliegenden Erfindung ist, dass sich durch dieses Verfahren ein kleines Verhältnis von Gesamt- zu Kernvolumen und eine hohe Konzentration von enkapsulierten biologischen Zellen im Verhältnis zum Gesamttransplantatvolumen ergibt. Vorteilsmäßig kann durch Variation der Kanalgeometrie der Durchmesser des Kerns, sowie die Dicke der äußeren Hüllschicht nahezu beliebig variiert werden.

Alle aus dem Stand der Technik bekannten Verkapselungsmaterialien können zur Bildung des Kerns und zum Aufbau der äußeren Hüllschicht zur Verwendung kommen. Vorzugsweise werden gereinigte Alginate (z.B. nach DE 198 36 960) verwendet. Wobei Alginate mit einer mittleren Molmasse von 20 kDa bis 10.000 kDa verwendet werden können, vorzugsweise liegt die Molmasse zwischen 100 kDa und 1200 kDa. Die Viskosität einer 0,1 %igen (w/v) hergestellten wässrigen Alginatlösung des zu verwendenden Alginates kann zwischen 3 und 100 mPa s liegen, vorzugsweise liegt sie zwischen 10 und 60 mPa s. Die Konzentration des Alginates zur Herstellung der zu verwendenden Alginatlösung für die Bildung der einschichtigen Kapseln und der Hüllschicht liegt zwischen 0,1 und 4% (w/v), vorzugsweise liegt sie zwischen 0,4 und 1% (w/v). Für die einschichtigen Kapseln und für die Hüllschicht können unterschiedliche Alginatkonzentrationen gewählt werden.

Zur Herstellung der vorgeschlagenen Mikrokapseln wird zunächst in einem ersten Verfahrensschritt die einschichtige Mikrokapsel aus vernetztem Alginat mit biologische Zellen geformt. Hierzu wird zunächst eine Mischung (Suspension) aus der löslichen Form des Alginats (z.B. Kalium- oder Natriumalginat in physiologischer Kochsalzlösung) und den biologischen Zellen hergestellt in einer Konzentration von bis zu 5*10⁷ Zellen pro ml Alginatlösung. Im Fall der Verkapselung von Gewebepartikeln anstatt von einzelnen biologischen Zellen kann die Konzentration wesentlich geringer gewählt werden.

Die homogene Zell/Alginat Suspension wird durch eine luftbeaufschlagte Sprühdüse gedrückt, welche aus drei Kanälen aufgebaut ist: Einem inneren Kanal, einer äußeren Düse und einem äußeren Luftring. Für den inneren Kanal werden vorzugsweise Kanülen mit einem Innendurchmesser von 50 µm bis 2000 µm verwendet. Die äußere Düse weist einen Innendurchmesser von 60 µm bis 4000 µm auf, der äußere Luftring hat vorzugsweise einen Innendurchmesser von 100 µm bis 5000 µm. Im ersten Verfahrenschritt zur Herstellung der einschichtig aufgebauten Mikrokapseln, werden nur der innere Kanal und der äußere Luftring benötigt. Deshalb kann für diesen Schritt auch eine entsprechend aufgebaute Sprühdüse bestehend aus zwei Kanälen verwendet werden. Durch die innere Düse findet bei der Verwendung einer dreikanäligen Sprühdüse kein Materialfluss statt. Die Suspension wird mit einer Geschwindigkeit von 10 µl/min bis 5 ml/min durch den inneren Kanal gedrückt, so dass sich am unteren Ausgang der Kanüle Tropfen formen, die aufgrund des Luftstromes, der durch den äußeren Luftring mit einer Geschwindigkeit von 0,5 l/min bis 10 l/min zugeführt wird, abreißen. Die biologische Zellen enthaltenen Tropfen fallen in eine den Vernetzer enthaltene Lösung, welche sich in einem Abstand von 4 cm bis 60 cm unter dem unteren Ende der Sprühdüse befindet. Während des Falles rundet sich der Tropfen zu einer sphärischen geometrischen Form ab. Die vernetzerhaltige Lösung besteht vorzugsweise aus zweiwertigen Kationen, z.B. gelöstes Calcium oder Barium (5-100 mM) oder anderen zwei- oder mehrwertige Kationen. Zusätzlich enthält das Fällbad vorzugsweise auch eine Puffersubstanz (z.B. Histidin 1 mM-10mM) und Kochsalz (z.B. 290 mOsmol).

Die Vernetzer bewirken eine ionische Vernetzung der Polymere und es entstehen somit einschichtig aufgebaute, wasserunlösliche Mikrokapseln mit einer Größe von 50 µm bis 3000 µm, Der Durchmesser der Mikrokapseln ist abhängig von der gewählten Größe und Geometrie der verwendeten Kanäle. Nach der Vertropfung folgen vorzugsweise mehrere Waschschritte der Mikrokapseln mit einer physiologischen Kochsalzlösung oder einer anderen geeigneten Waschlösung und gegebenenfalls eine Inkubation in einer Natriumsulfatlösung vorzugsweise nach US 6592886. Die Abtrennung der Mikrokapseln aus dem Fällungs- und Waschbädern erfolgt vorzugsweise mit einer Zentrifuge oder anderen geeigneten Methoden.

Im zweiten Verfahrensschritt werden die einschichtigen Mikrokapseln welche im ersten Verfahrensschritt hergestellt wurden in einer Polymerlösung vorzugsweise einer 0,1 %igen bis 4%igen (w/v) Alginatlösung aufgenommen. Dieses Gemisch wird wiederum durch den inneren Kanal der oben beschriebenen luftbeaufschlagten Sprühdüse mit einer Geschwindigkeit von 10 µl/min bis 5 ml/min gedrückt. Durch den zweiten Kanal, der inneren Düse, wird in diesem Verfahrensschritt gleichzeitig eine reine Polymerlösung ohne biologische Zellen, vorzugsweise eine 0,1 %ige bis 4%ige (w/v) Alginatlösung mit einer Geschwindigkeit von 10 µl/min bis 5 ml/min gedrückt. Am Ende der Düse bilden sich dadurch zweischichtige Tropfen, die aufgrund des Luftstromes, der durch den äußeren Luftring mit einer Geschwindigkeit von 0,5 l/min bis 10 l/min zugeführt wird abreißen. Durch diesen Schritt wird auf die einschichtigen Mikrokapseln eine Hüllschicht aus dem verwendeten Polymer aufgetragen. Die Geschwindigkeiten mit denen die Lösungen durch den inneren und den äußeren Kanal gedrückt werden können sich voneinander unterscheiden. Die Polymerkonzentrationen der einschichtigen Mikrokapseln, der Polymerlösung in der diese Mikrokapseln aufgenommen werden und die Polymerkonzentration aus der die äußere Hüllschicht besteht können sich ebenfalls voneinander unterscheiden. Die biologische Zellen enthaltenen Tropfen fallen in ein den Vernetzer enthaltene Lösung, welche sich in einem Abstand von 4 cm bis 60 cm unter dem unteren Ende der Sprühdüse befindet. Während des Falles runden sich die Tropfen zu einer sphärischen geometrischen Form ab. Die vernetzerhaltige Lösung besteht, falls Alginate verwendet wurden, vorzugsweise aus zweiwertigen Kationen, z.B. gelöstes Calcium oder Barium (5-100 mM) oder anderen zwei- oder mehrwertige Kationen. Zusätzlich enthält das Fällbad vorzugsweise auch eine Puffersubstanz (z.B. Histidin) und Kochsalz (z.B. 290 mOsmol). Falls andere Polymere als Alginate verwendet werden, sind dem Stand der Technik entsprechende Vernetzersubstanzen und Puffersubstanzen zu verwenden. Die Vernetzer bewirken wie in dem ersten Verfahrensschritt eine ionische Vernetzung der Polymere und es entstehen somit zweischichtig aufgebaute, wasserunlösliche Mikrokapseln mit einer Größe von 60 µm bis 4000 µm. Der Durchmesser der zweischichtig aufgebauten Mikrokapseln ist abhängig von der gewählten Größe und Geometrie der verwendeten Kanäle. Nach der Vertropfung folgen vorzugsweise mehrere Waschschritte der Mikrokapseln mit einer physiologischen Kochsalzlösung oder einer anderen geeigneten Waschlösung und gegebenenfalls eine Inkubation in einer Natriumsulfatlösung vorzugsweise nach US 6592886. Die Abtrennung der Mikrokapseln aus dem Fällungs- und Waschbädern erfolgt vorzugsweise mit einer Zentrifuge oder anderen geeigneten Methoden.

Der zweite Arbeitsschritt kann beliebig oft wiederholt werden, so dass mehrschichtige Kapseln hergestellt werden können.

Zur Anwendung können alle bekannten dem Stand der Technik entsprechenden polymeren Biomaterialien kommen z.B. aus dem Bereich der natürlichen Polymere: Proteine oder auf Proteine basierende Polymere (z.B. Kollagene, Albumine u.a.), Polyaminosäuren (z.B. Poly α-L-Lysine, Poly-L-Glutaminsäure u.a.), Polysaccacharide und deren Derivate (z.B. Carboxymethylcellulose, Cellulosesulfat, Agarose, Alginate, Carrageenane, Hyaluronsäure, Heparin und Heparin ähnliche Glukosaminosulfate, Dextrane und seine Derivate, Chitosan und seine Derivate). Verwendet werden können auch polymere Biomaterialien aus dem Bereich der synthetischen Polymere: aliphatische Polyester (z.B. Polylactidsäure, Polyglycolsäure, Polyhydroxybutyrate u.a.), Polyamide, Polyanhydride, Polyorthoester, Polyphosphazene, thermoplastische Polyurethane, Polyvinylalkohole, Polyhydroxyethyl methacrylate, Polymethyl methacrylate und Polytetrafluoroethylene.

### Beispiele

### 1. Herstellung von zweischichtigen Mikrokapseln mit biologischen Zellen (Konzentration 2 x 10⁷ Zellen /ml) und einem Durchmesser der inneren Mikrokapsel von ca. 400 µm und einer Hüllschicht mit einer Dicke von ca. 200 µm.

Die kultivierten zu verkapselnden biologischen Zellen werden mit PBS (PAA, Österreich) gewaschen und mit Trypsin/EDTA (PAA, Österreich) abgelöst. Die Reaktion wird zügig mit Medium (abhängig vom Zelltyp, z.B. RPMI, PAA, Österreich) abgestoppt und die Zellsuspension abzentrifugiert (8 min bei 1200 rpm). Das Pellet wird in PBS resuspendiert und die Zellzahl wird bestimmt. Die gewünschte Zellmenge von 1,4x10⁷ Zellen wird erneut abzentrifugiert (8 min bei 1200 rpm). Anschließend wird das PBS vollständig abgesaugt und 60 µl Pellet luftblasenfrei in 80 µl PBS resuspendiert. Diese Zellsuspension wird in 560 µl einer 0,8 % igen (w/v) Kaliumalginatlösung (Verwendet wurde ein Alginat mit einer Viskosität von ca. 40 mPa·s einer 0,1 %igen (w/v) wässrigen Lösung) aufgenommen.

Um die resuspendierten Zellen mit der Alginatlösung zu vermischen wird diese in eine 1 ml Spritze mit Kanüle aufgezogen und durch mehrmaliges langsames Aufund Abziehen homogen mit den Zellen vermischt. Es entsteht eine Zellkonzentration von 2 x 10⁷ Zellen /ml

Für die Herstellung der einschichtig aufgebauten Mikrokapseln mit einem Durchmesser von 400 µm wird in der luftbeaufschlagten dreikanaligen Sprühdüse für den inneren Kanal eine Kanüle mit einem Innendurchmesser von 400 µm verwendet. Die Kanüle wird in einer äußeren Düse mit einem Innendurchmesser von 700 µm fixiert. Ein Luftring mit einer Öffnung von 1,5 mm wird über die beiden inneren Kanäle geschraubt. Das homogene Zell/Alginatlösungs-Gemisch wird durch die beschriebene Sprühdüse vertropft. Dazu wird die, das Gemisch enthaltene 1 ml Spritze mittels eines Lueranschlusses auf den inneren Kanal gesetzt. Das Zell/Alginatlösungs-Gemisch wird mit einer Geschwindigkeit von 300 µl/min durch den innneren Kanal gedrückt. Der Luftstrom wird durch den äußeren Luftring mit einer Geschwindigkeit von 2,5 l/min geleitet. Die entstehenden Mikrokapseln fallen in ein bariumhaltiges Fällbad (20 mM BaCl, 5 mM L-Histidin, 124 mM NaCl, pH 7,0 ± 0,1, 290 mOsmol ± 3) welches ca. 10 cm unterhalb der Sprühdüse aufgebaut ist. Nach einer Verweilzeit von 5 min in dem bariumhaltigem Fällbad werden die Mikrokapseln 5mal mit jeweils 20ml PBS gewaschen.

500 µl der einschichtig aufgebauten Mikrokapseln werden anschließend in 500 µl einer 0,8 %igen (w/v) Alginatlösung (Verwendet wurde ein Alginat mit einer Viskosität von ca. 40 mPa·s einer 0,1 %igen (w/v) wässrigen Lösung) aufgenommen und homogen vermischt. Diese Suspension wird in einer 1 ml Spritze aufgenommen und mittels eines Lueranschlußes an den inneren Kanal (Innendurchmesser: 400 µm) der Sprühdüse angeschlossen und mit einer Geschwindigkeit von 50 µl/min durch diesen gedrückt. An den zweiten inneren Kanal (Innendurchmesser: 700 µm) wird eine 5 ml Spritze mit einer 0,8 %igen Alginatlösung mittels eines Lueranschluß angeschlossen und mit einer Geschwindigkeit von 250 µl/min durch diesen gedrückt. Der Luftstrom wird durch den äußeren Luftring mit einer Geschwindigkeit von 2,9 l/min geleitet. Die entstehenden Mikrokapseln fallen in ein bariumhaltiges Fällbad (20 mM BaCl, 5 mM L-Histidin, 124 mM NaCl, pH 7,0 ± 0,1, 290 mOsmol ± 3) welches ca. 10 cm unterhalb der Sprühdüse aufgebaut ist. Nach einer Verweilzeit von 5 min in dem bariumhaltigem Fällbad werden die Mikrokapseln 4mal mit jeweils 20ml PBS gewaschen und einmal mit Medium. Durch diesen Prozess entstehen zweischichtig aufgebaute Mikrokapseln mit einem Gesamtdurchmesser von ca. 800µm.

### 2. Herstellung von zweischichtigen Mikrokapseln mit biologischen Zellen (Konzentration 2 x 10⁶ Zellen /ml) und einem Durchmesser der inneren Mikrokapsel von ca. 1000 µm und einer Hüllschicht mit einer Dicke von 400 µm.

Die kultivierten zu verkapselnden biologischen Zellen werden mit PBS (PAA, Österreich) gewaschen und mit Trypsin/EDTA (PAA, Österreich) abgelöst. Die Reaktion wird zügig mit Medium (abhängig vom Zelltyp, z.B. RPMI, PAA, Österreich) abgestoppt und die Zellsuspension abzentrifugiert (8 min bei 1200 rpm). Das Pellet wird in PBS resuspendiert und die Zellzahl wird bestimmt. Die gewünschte Zellmenge von 2 x10⁶ Zellen wird erneut abzentrifugiert (8 min bei 1200 rpm). Anschließend wird das PBS vollständig abgesaugt und 50 µl Pellet luftblasenfrei in 150 µl PBS resuspendiert. Diese Zellsuspension wird in 800 µl einer 0,6 % igen (w/v) Kaliumalginatlösung (Verwendet wurde ein Alginat mit einer Viskosität von ca. 40 mPa·s einer 0,1 %igen (w/v) wässrigen Lösung) aufgenommen.

Um die resuspendierten Zellen mit der Alginatlösung zu vermischen wird diese in eine 1 ml Spritze mit Kanüle aufgezogen und durch mehrmaliges langsames Aufund Abziehen homogen mit den Zellen vermischt. Es entsteht eine Zellkonzentration von 2 x 10⁶ Zellen /ml

Für die Herstellung der einschichtig aufgebauten Mikrokapseln mit einem Durchmesser von ca. 1000 µm wird in der luftbeaufschlagten dreikanaligen Sprühdüse für den inneren Kanal eine Kanüle mit einem Innendurchmesser von 800 µm verwendet. Die Kanüle wird in einer äußeren Düse mit einem Innendurchmesser von 1200 µm fixiert. Ein Luftring mit einer Öffnung von 2,0 mm wird über die beiden inneren Kanäle geschraubt. Das homogene Zell/Alginatlösungs-Gemisch wird durch die beschriebene Sprühdüse vertropft. Dazu wird die, das Gemisch enthaltene 1 ml Spritze mittels eines Lueranschlusses auf den inneren Kanal gesetzt. Das Zell/Alginatlösungs-Gemisch wird mit einer Geschwindigkeit von 200 µl/min durch den innneren Kanal gedrückt. Der Luftstrom wird durch den äußeren Luftring mit einer Geschwindigkeit von 2,4 l/min geleitet. Die entstehenden Mikrokapseln fallen in ein bariumhaltiges Fällbad (20 mM BaCl, 5 mM L-Histidin, 124 mM NaCl, pH 7,0 ± 0,1, 290 mOsmol ± 3) welches ca. 10 cm unterhalb der Sprühdüse aufgebaut ist. Nach einer Verweilzeit von 5 min in dem bariumhaltigem Fällbad werden die Mikrokapseln 5mal mit jeweils 20ml PBS gewaschen.

1000 µl der einschichtig aufgebauten Mikrokapseln werden anschließend in 1000 µl einer 0,8 %igen (w/v) Alginatlösung (Verwendet wurde ein Alginat mit einer Viskosität von ca. 40 mPa·s einer 0,1 %igen (w/v) wässrigen Lösung) aufgenommen und homogen vermischt. Diese Suspension wird in einer 2 ml Spritze aufgenommen und mittels eines Lueranschlußes an den inneren Kanal (Innendurchmesser: 1000 µm) der Sprühdüse angeschlossen und mit einer Geschwindigkeit von 200 µl/min durch diesen gedrückt. An den zweiten inneren Kanal (Innendurchmesser: 1200 µm) wird eine 10 ml Spritze mit einer 0,6 %igen Alginatlösung mittels eines Lueranschluß angeschlossen und mit einer Geschwindigkeit von 1000 µl/min durch diesen gedrückt. Der Luftstrom wird durch den äußeren Luftring mit einer Geschwindigkeit von 3,4 l/min geleitet. Die entstehenden Mikrokapseln fallen in ein bariumhaltiges Fällbad (20 mM BaCl, 5 mM L-Histidin, 124 mM NaCl, pH 7,0 ± 0,1, 290 mOsmol ± 3) welches ca. 10 cm unterhalb der Sprühdüse aufgebaut ist. Nach einer Verweilzeit von 5 min in dem bariumhaltigem Fällbad werden die Mikrokapseln 4mal mit jeweils 20ml PBS gewaschen und einmal mit Medium. Durch diesen Prozess entstehen zweischichtig aufgebaute Mikrokapseln mit einem Gesamtdurchmesser von ca. 1800µm.

### 3. Erstellung von zweischichtigen Mikrokapseln mit biologischen Gewebestücken (sekundäres hyperplastisches Nebenschilddrüsengewebe) und einem Durchmesser der inneren Mikrokapsel von ca. 800 µm und einer Hüllschicht mit einer Dicke von 400 µm.

Das humane hyperplastische Nebenschildrüsengewebe wird mit einem Skalpell in Gewebepartikel (Durchmesser: ca. 700µm) zerteilt. Die Gewebepartikel werden fünf mal für jeweils zwei Minuten mit jeweils 30ml PBS. Anschließend werden die Stücke in einer 0,5% igen (w/v) Kaliumalginatlösung (Verwendet wurde ein Alginat mit einer Viskosität von ca. 40 mPa·s einer 0,1 %igen (w/v) wässrigen Lösung) aufgenommen. Die gewaschenen Gewebepartikel werden in der Alginatlösung suspendiert und in einer 1 ml Spritze aufgenommen.

Für die Herstellung der einschichtig aufgebauten Mikrokapseln mit einem Durchmesser von ca. 800 µm wird in der luftbeaufschlagten dreikanaligen Sprühdüse für den inneren Kanal eine Kanüle mit einem Innendurchmesser von 700 µm verwendet. Die Kanüle wird in einer äußeren Düse mit einem Innendurchmesser von 1500 µm fixiert. Ein Luftring mit einer Öffnung von 3,0 mm wird über die beiden inneren Kanäle geschraubt. Das homogene Gewebe/Alginatlösungs-Gemisch wird durch die beschriebene Sprühdüse vertropft. Dazu wird die, das Gemisch enthaltene 1 ml Spritze mittels eines Lueranschlusses auf den inneren Kanal gesetzt. Das Zell/Alginatlösungs-Gemisch wird mit einer Geschwindigkeit von 500 µl/min durch den innneren Kanal gedrückt. Der Luftstrom wird durch den äußeren Luftring mit einer Geschwindigkeit von 5,0 l/min geleitet. Die entstehenden Mikrokapseln fallen in ein bariumhaltiges Fällbad (20 mM BaCl, 5 mM L-Histidin, 124 mM NaCl, pH 7,0 ± 0,1, 290 mOsmol ± 3) welches ca. 10 cm unterhalb der Sprühdüse aufgebaut ist. Nach einer Verweilzeit von 5 min in dem bariumhaltigem Fällbad werden die Mikrokapseln 5mal mit jeweils 20ml PBS gewaschen.

Die in der PBS Lösung liegenden gewebehaltigen einschichtigen Mikrokapseln werden erneut in einer 1ml Spritze aufgenommen und in eine neue Petrischale überführt. Die PBS Lösung wird vollständig abgezogen und die einschichtig aufgebauten Mikrokapseln werden in 500 µl einer 0,5%igen (w/v) Alginatlösung aufgenommen und homogen vermischt. Diese Suspension wird in einer 1ml Spritze aufgezogen und mittels eines Lueranschlußes an den inneren Kanal (Innendurchmesser: 800 µm) der Sprühdüse angeschlossen und mit einer Geschwindigkeit von 200 µl/min durch diesen gedrückt. An den zweiten inneren Kanal (Innendurchmesser: 1500 µm) wird eine 5ml Spritze mit einer 0,5 %igen Alginatlösung (Verwendet wurde ein Alginat mit einer Viskosität von ca. 40 mPa·s einer 0,1 %igen (w/v) wässrigen Lösung) mittels eines Lueranschluß angeschlossen und mit einer Geschwindigkeit von 1000 µl/min durch diesen gedrückt. Der Luftstrom wird durch den äußeren Luftring mit einer Geschwindigkeit von 4,0 l/min geleitet. Die entstehenden Mikrokapseln fallen in ein bariumhaltiges Fällbad (20 mM BaCl, 5 mM L-Histidin, 124 mM NaCl, pH 7,0 ± 0,1, 290 mOsmol ± 3) welches ca. 10 cm unterhalb der Sprühdüse aufgebaut ist. Nach einer Verweilzeit von 5 min in dem bariumhaltigem Fällbad werden die Mikrokapseln 4mal mit jeweils 20ml PBS gewaschen und einmal mit Medium. Es entstehen gewebehaltige zweischichtig aufgebaute Mikrokapseln mit einem Gesamtdurchmesser von 1600 µm.

## Patentansprüche

1. Verfahren zur Herstellung zwel- oder mehrschichtig aufgebauter sphärischer Mikrokapseln, bestehend aus einer inneren Mikrokapsel aus vernetztem Alginat und biologischen Zellen und einer oder mehreren Schichten aus vernetzten Polymeren ohne biologische Zellen, die die innere Mikrokapsel mit den biologischen Zellen vollständig umhüllt bzw. umhüllen, unter Verwendung einer dreikanäligen luftbeaufschlagten Spruhdüse mit einem inneren Kanal, einer äußeren Düse und einem äußeren Lüftring, wobei in einem ersten Verfahrensschritt einschichtig aufgebaute Mikrokapseln aus vernetztem Alginat mit biologischen Zellen hergestellt werden, indem eine homogene Zell/Alginat-Suspension durch den inneren Kanal der dreikanaligen Sprühdüse gedrückt wird, und in mindestens einem weiteren Verfahrensschritt auf den einschichtig aufgebauten Mikrokapseln mindestens eine äußere Hüllschicht aus vernetzten Polymeren aufgetragen wird, die keine biologischen Zellen enthält, indem die im ersten Verfahrenschritt hergestellten einschichtigen Mikrokapseln in Polymer-Lösung wiederum durch den inneren Kanal der dreikanäligen Sprühdüse gedrückt werden und gleichzeitig eine Polymer-Lösung ohne biologische Zellen durch den zweiten Kanal der dreikanäligen Sprühdüse gedrückt wird, wobei für die innere Mikrokapsel und die mindestens eine äußere Hüllschicht ein chemisch identisches Polymer oder chemisch unterschiedliche Polymere in identischer oder unterschiedlichen Konzentrationen verwendet werden, wobei außerdem die Polymeren unterschiedliche Molmassen haben und/oder unterschiedlich vernetzt sein können.

2. Verfahren nach Anspruch 1, in dem anstelle von biologischen Zellen Gewebepartikel menschlichen oder tierischen Ursprungs verwendet werden oder biologische Zellen und Gewebepartikel gleichzeitig verwendet werden.

3. Verfahren nach einem der Ansprüche 1 oder 2, in dem der Durchmesser der inneren Kapsel eine Größe von 10 bis 2000 µm aufweist und die äußere Hüllschicht eine Dicke von 10 bis 2000 µm aufweist.

4. Verfahren nach einem der Ansprüche 1 bis 3, in dem mehrere Hüllschichten aufgetragen werden und die inneren Hüllschichten biologische Zellen und/oder Gewebepartikel enthalten, die sich gegebenenfalls von den biologischen Zellen und/oder Gewebepartikeln in der inneren Mikrokapsel unterscheiden können.

5. Verfahren nach einem der Ansprüche 1 bis 4, in dem die mehrschichtigen Mikrokapseln vor oder nach dem Auftragen oder äußeren Hüllschicht mit einem Polykation umgeben werden.

6. Verfahren nach Anspruch 5, in dem das Polykation Poly-L-Lysin ist.

7. Verfahren nach einem der. Ansprüche 1 bis 6, in dem anstelle von biologischen Zellen Substanzen verwendet werden.

8. Verfahren nach Anspruch 7, in dem die Substanzen unter Therapeutika, Zytostatika und Nahrungsergänzungsmitteln ausgewählt werden.

9. Verfahren nach Anspruch 8, in dem die Therapeutika unter Proteinen und Hormonen, die Zytostatika unter Proteinen und Angiogenesehemmem und die Nahrungsergänzungsmittel unter Vitaminen und ungesättigten Fettsäuren ausgewählt werden.

## Claims

1. A method for producing spherical microcapsules with a double- or multiple-layer structure, consisting of an inner microcapsule made of cross-linked alginate and biological cells and one or more layers of cross-linked polymers without any biological cells which fully enclose(s) the inner microcapsule with the biological cells, using a three-channel air-charged spray nozzle with an inner channel, an outer nozzle and an outer air ring, wherein in a first method step microcapsules with a single-layer structure are produced from cross-linked alginate with biological cells, by a homogeneous cell/alginate suspension being pushed through the inner channel of the three-channel spray nozzle, and wherein in at least one further method step at least one outer covering layer made of cross-linked polymers and which does not contain any biological cells being applied to the microcapsules with a single-layer structure, by pushing the single-layered microcapsules produced in the first method step in a polymer solution again through the inner channel of the three-channel spray nozzle, and at the same time by pushing a polymer solution without any biological cells being pushed through the second channel of the three-channel spray nozzle, whereby a chemically identical polymer or chemically different polymers in identical or different concentrations are used for the inner microcapsule and the at least one outer covering layer, wherein, additionally, the polymers may have different molar masses and/or may be cross-linked in different ways.

2. The method according to claim 1, wherein, instead of biological cells, tissue particles of human or animal origin are used, or biological cells and tissue particles are used at the same time.

3. The method according to any one of claims 1 or 2, wherein the diameter of the inner capsule is 10 to 2000 µm in size, and the outer covering layer has a thickness of from 10 to 2000 µm.

4. The method according to any one of claims 1 to 3, wherein several covering layers are applied and the inner covering layers contain biological cells and/or tissue particles which, if appropriate, can be different from the biological cells and/or tissue particles in the inner microcapsule.

5. The method according to any one of claims 1 to 4, wherein the multiple-layered microcapsules are enclosed with a polycation before or after application of the outer covering layer.

6. The method according to claim 5, wherein the polycation is poly-L-lysine.

7. The method according to any one of claims 1 to 6, wherein, instead of biological cells, substances are used.

8. The method according to claim 7, wherein the substances are selected from therapeutic agents, cytostatic drugs and dietary supplements.

9. The method according to claim 8, wherein the therapeutic agents are selected from proteins and hormones, the cytostatic drugs are selected from proteins and angiogenesis inhibitors, and the dietary supplements are selected from vitamins and unsaturated fatty acids.

## Revendications

1. Procédé de préparation de microcapsules sphériques à deux ou plusieurs couches, consistant en une microcapsule interne d'alginate réticulé et de cellules biologiques, et une ou plusieurs couches de polymères réticulés sans cellules biologiques, qui enveloppent totalement la microcapsule interne avec les cellules biologiques, en utilisant un pulvérisateur à alimentation d'air, à trois canaux, avec un canal interne, une buse externe et un anneau d'air externe, dans lequel on prépare, dans une première étape du procédé, des microcapsules avec une seule couche d'alginate réticulé avec des cellules biologiques, en en envoyant une suspension homogène cellules/alginate dans le canal interne du pulvérisateur à trois canaux, et on applique sur les microcapsules à une couche, dans au moins une autre étape de procédé, au moins une couche d'enveloppe externe en polymère réticulé qui ne contient pas de cellules biologiques, en envoyant les microcapsules avec une seule couche, préparées dans la première étape de procédé, dans une solution de polymère à nouveau dans le canal interne du pulvérisateur à trois canaux, et en envoyant simultanément une solution de polymère sans cellules biologiques dans le deuxième canal du pulvérisateur à trois canaux, où l'on utilise pour la microcapsule interne et la au moins une couche d'enveloppe extérieure, des polymères de même nature chimique ou des polymères chimiquement différents en concentrations identiques ou différentes, les polymères pouvant en outre avoir des masses molaires différentes et/ou pouvant être réticulés différemment.

2. Procédé selon la revendication 1, dans lequel à la place des cellules biologiques, on utilise des particules de tissu d'origine humaine ou animale ou on utilise simultanément des cellules biologiques et des particules de tissu.

3. Procédé selon l'une des revendications 1 ou 2, dans lequel le diamètre de la capsule interne a une taille allant de 10 à 2000 µm et la couche d'enveloppe externe a une épaisseur allant de 10 à 2000 µm.

4. Procédé selon l'une des revendications 1 à 3, dans lequel plusieurs couches d'enveloppe sont appliquées et les couches d'enveloppe internes contiennent des cellules biologiques et/ou des particules de tissu, qui peuvent être différents des cellules biologiques et/ou particules de tissu de la microcapsule interne.

5. Procédé selon l'une des revendications 1 à 4, dans lequel les microcapsules à plusieurs couches sont entourées, avant ou après l'application de la couche d'enveloppe externe, d'un polycation.

6. Procédé selon la revendication 5, dans lequel le polycation est une poly-L-lysine.

7. Procédé selon l'une des revendications 1 à 6, dans lequel on utilise des substances à la place des cellules biologiques.

8. Procédé selon la revendication 7, dans lequel les substances sont choisies parmi des agents thérapeutiques, des cytostatiques et des compléments alimentaires.

9. Procédé selon la revendication 8, dans lequel les agents thérapeutiques sont choisis parmi les protéines et les hormones, les cytostatiques, parmi les protéines et les inhibiteurs d'angiogenèse, et les compléments alimentaires, parmi les vitamines et les acides gras insaturés.
